# EUROPÄISCHE PATENTSCHRIFT

(11) **EP 1 388 322 B1**
(45) Veröffentlichungstag und Bekanntmachung des Hinweises auf die Patenterteilung: **10.06.2009**
(21) Anmeldenummer: 02017736.6
(22) Anmeldetag: 08.08.2002
(51) Int. Cl.: A61B 6/08, A61B 6/12, A61B 6/04, A61B 5/06

(54) **System zur Patientenpositionierung für die Strahlentherapie/Radiochirurgie basierend auf magnetischem Tracking eines Implantats**
System for patient positioning in radiationtherapy / radiosurgery based on magnetic tracking of an implant
Système de positionnement d'un patient pour la radiothérapie / radiochirurgie basée sur des systèmes de suivi magnétique d'un implant

(43) Veröffentlichungstag der Anmeldung: 11.02.2004
(73) Patentinhaber: BrainLAB AG, 85622 Feldkirchen (DE)
(72) Erfinder: Vilsmeier, Stefan, 6330 Kufstein (AT); Fröhlich, Stephan, 85609 Aschheim (DE); Erbel, Stephan, 81677 München (DE)
(74) Vertreter: Schwabe - Sandmair - Marx

(56) Entgegenhaltungen:
- DE-A- 19 856 467
- DE-A- 19 953 177
- US-A- 5 394 875
- US-A- 5 538 494
- US-A- 5 769 861
- US-A1- 2002 085 668
- US-A1- 2002 091 314

## Beschreibung

Die vorliegende Erfindung betrifft ein Verfahren bzw. ein System zur exakten Positionierung eines Patienten in der Strahlentherapie bzw. Radiochirurgie bzw. zur Berücksichtigung von atembedingten Verschiebungen interner Strukturen des Patienten sowohl bei der eigentlichen Behandlung, als auch bei der Aufnahmen der dafür notwendigen Bilddaten.

In der Strahlentherapie und der Radiochirurgie wurden in letzter Zeit große Fortschritte in der Dosisplanung erzielt. Es wird angestrebt mit höheren Strahlendosen zu arbeiten, welche möglichst präzise unter Schonung der umgebenden Regionen auf ein Zielvolumen, also beispielsweise auf einen Tumor, appliziert werden. Obwohl die Dosisplanung, wie erwähnt, relativ gute Erfolge zeigt, steht der Verwendung von höheren Dosen, die in u.U. sogar in einer einzigen oder in wenigen Fraktionen verabreicht werden, oftmals die Tatsache im Wege, dass der Patient bzw. der zu bestrahlende Körperabschnitt, nur relativ ungenau positioniert werden kann. Um größere Schädigungen des gesunden Gewebes zu vermeiden, ist eine exakte Positionierung essentiell.

Auch eine optimale Positionierung des Patienten kann unter Umständen nicht ausreichend sein, wenn das Zielgewebe welches bestrahlt werden soll sich im Patienten bewegt. Vor allem die Atembewegung kann zu einer Verschiebung von bis zu etwa 2 cm führen. Um eine optimale Behandlung auch in diesen Fällen gewährleisten zu können ist es notwendig die genaue Position des Zielgebietes im Patienten zu jedem Zeitpunkt der Behandlung zu kennen. Ist diese Position bekannt, so ist es möglich das Therapiegerät nur zu den Zeiten zu aktivieren, zu denen das Zielvolumen im Patienten innerhalb eines Toleranzbereiches um das Zielgebiet des Therapiegerätes liegt, oder, sofern mit der jeweiligen Bauart der Maschine zu vereinbaren, das Zielgebiet der Therapiemaschine mit der Bewegung des Ziels mitzuführen.

Ein verwandtes Problem stellt sich bei der Aufnahmen der diagnostischen Bilder auf denen die Behandlungen basieren. Bewegt sich das Innere des Patienten während der Aufnahme dieser Bilddaten, so entstehen Artefakte in den Bildern und die geometrischen Proportionen des Inneren des Patienten werden verzerrt. Das Zielvolumen im Patienten wird dementsprechend nicht in seiner wahren Größe erfasst, sondern kann zu groß oder zu klein dargestellt werden.

Des Weiteren erfassen gängige röntgenbasierende Verfahren meistens nur die knöchernen Strukturen des Patienten und sind so für die Behandlung von Organen welche sich relativ zu diesen knöchernen Strukturen verschieben können (zum Beispiel Prostata) relativ ungeeignet. Ein weiterer Nachteil der röntgenbasierenden Verfahren ist die limitierte zeitliche Auflösung, sowie die entstehende Strahlenbelastung, bei der Verfolgung atembedingter Verschiebungen über einen längeren Zeitraum. Die Kosten für stereoskopische Röntgensystem sind für viele Anwendungen ein Hindernis.

Aus der US-A-6 230 038 ist ein System zur therapeutischen Strahlenbehandlung eines Patienten bekannt, bei dem ein magnetisches Element zur Lokalisierung eines Behandlungsortes Verwendung findet. Dieses magnetische Element wird extern durch ein Magnetfeld beaufschlagt und das wiederum vom magnetischen Element emittierte Signal zur Lokalisierung des magnetischen Elements durch einen Empfänger mit daran angeschlossener Recheneinheit empfangen. So lässt sich die Behandlung auf eine unmittelbare Umgebung des magnetischen Elements und des daran angrenzenden Behandlungszielortes beschränken.

Aus der US-A-2002/0091314 ist ein Verfahren bekannt, durch welches bei einer Strahlentherapie die Atmungsbewegung des Patienten kompensiert werden kann. Dabei wird die Bewegung des Zielortes für die Behandlung durch im Bereich des Behandlungszielortes implantierte Marker bestimmt. Diese Marker können Spulen sein, die durch ein externes Magnetfeld beaufschlagt werden. Das dadurch durch die Spulen emittierte Signal wird aufgenommen und dient zur Lokalisierung des Behandlungszielortes in Echtzeit.

Aus der "Real Time Tracking of Tumor Positions for Precision Irradiation", CARS, S. Kirsch, Ch. Schilling, 1998 ist ein Verfahren zur Behandlung sich bewegender Tumore bekannt, wobei eine Markerspule im Bereich des Tumors implantiert wird und sich demzufolge mit ihm bewegt. Falls sich die lokalisierte Markerspule zusammen mit dem Tumor aus dem fixen Bestrahlungsfokus, beispielsweise durch Atmung bewegt, so kann die Bestrahlung ausgesetzt werden.

Aus der WO-A-96/08999 ist ein Verfahren und eine Vorrichtung bekannt, bei dem ein magnetisches Implantat an einen Behandlungszielort anliegend angeordnet wird und durch einen positionell bestimmbaren Sensor verfolgt wird.

Aus dem Stand der Technik sind lediglich Messpunkte bzw. Sensoren bekannt, die ortsfest im Behandlungsraum angeordnet sind und über die die Position der Implantate bestimmt werden kann. Dabei ist es von Nachteil, dass die Messsensoren bei Patienten mit unterschiedlichen Körpermaßen nicht optimal, d.h. so nahe wie möglich an den Patienten gebracht werden können. Daraus können Messungenauigkeiten aufgrund des Abstandes der Messsensoren von den Implantaten resultieren. Ferner würde die Anbringung des Messsystems nahe dem Patienten an einer ortsfesten Position die Bewegung des Beschleunigerkopfes für die Strahlentherapie des öfteren stören. Falls anstelle eines Beschleunigerkopfes für die Strahlentherapie eine Aufnahmevorrichtung für Bilddatensätze eingesetzt werden soll, gilt selbstverständlich analoges.

Es ist die Aufgabe der Erfindung, die oben genannten Probleme des Standes der Technik zu überwinden.

Diese Aufgabe wird durch Verfahren gemäß dem unabhängigen Anspruch 1 gelöst. Die Unteransprüche definieren dabei bevorzugte Ausführungsformen der Erfindung.

Die Erfindung kann alle in den Ansprüchen und in dieser Beschreibung genannten Merkmale einzeln oder in jedweder Kombination umfassen; sie beinhaltet ebenfalls eine Vorrichtung mit den Merkmalen, wie sie aus den Ansprüchen oder dieser Beschreibung entnehmbar sind sowie entsprechende Verwendungen und Computerprogramme bzw. Speichervorrichtungen hierfür.

### Beschreibung möglicher Ausführungsformen

### Allgemeine Beschreibung der Magnettechnik mit Implantaten

Die vorliegende Erfindung umgeht die oben genannten Probleme durch die Bestimmung der Position eines oder mehrerer Implantats (e) über ein magnetisches Trackingsystem. Das Problem der Energieversorgung des Implantats löst die Erfindung zum Beispiel durch eine induktive Übertragung elektromagnetischer Energie über ein dynamische Feld welches von Außen in den Patienten eingestrahlt wird. Auf diese Weise kann auf eine Verkabelung des Implantats sowie auf eine Energiespeichervorrichtung im Implantat verzichtet werden. Durch den Verzicht auf eine externe Verkabelung wird das Risiko einer Infektion für den Patienten sehr deutlich reduziert.

### Magnettracking mit Implantat zur Positionierung am Behandlungsgerät

Die aufgenommene elektromagnetische Energie wird vom Implantat zumindest teilweise wieder abgestrahlt, vorzugsweise in Form eines Wechselfeldes. Wird das vom Implantat abgestrahlte Signal in der Nähe des Patienten gemessen, so lässt sich daraus die Position des Implantats relativ zu dem oder den Messgeräten bestimmen. Magnetische Trackingsysteme sind aus der industriellen Anwendung bekannt. Im Gegensatz zu den meisten gängigen System wird bei der hier beschriebenen Erfindung die Position des Senders bestimmt. Die genaue Meßmethode ist für die Erfindung ohne besondere Bedeutung.

Ist die Position der Messpunkte relativ zum Therapiegerät bekannt, so kann damit auch die Position des Implantats relativ zum Therapiegerät bestimmt werden.

Die Erfindung soll im Folgenden am Beispiel eines konventionellen Linearbeschleunigers dessen Gantry sich um eine horizontale Achse drehen kann erläutert werden, kann aber genauso bei Linearbeschleunigern die auf Roboterarmen mit mehreren Freiheitsgrade geführt werden, oder auch bei radioaktivem Zerfall basierende Therapiesysteme angewandt werden.

Die Position der Messpunkte relativ zum Therapiegerät wird in Echtzeit über ein 6-dimensionales Trackingsystem (3 translatorische und 3 rotatorische Freiheitsgrade) bestimmt . Als Beispiel kann das ExacTrac™ -System der BrainLAB AG angeführt werden welches in der Lage ist die Position eines - mit einem oder mehreren Sensoren ausgestatteten - Messkopfes 6-dimensional zu verfolgen. Diese Lösung hat insbesondere den Vorteil, dass die Sensoren für die Messung sehr nah an den Patienten gebracht werden können um nach der Messung dann wieder entfernt zu werden. Je näher am Sender gemessen wird, desto weniger Leistung braucht das sendende Implantat und desto weniger wirken sich eventuelle Störfelder aus.

### Magnettracking mit Implantat zur atemgetriggerten Behandlung am Behandlungsgerät

Kann die Position des Implantats mehrmals in der Sekunde bestimmt werden, so kann eine eventuelle atembedingte Verschiebung des Implantats und damit auch die Verschiebung des Zielvolumens im Patienten bestimmt werden.

In Fällen in denen es eine negative Wechselwirkung zwischen dem Therapiegerät und dem magnetischen Trackingsystem gibt kann die gleiche Funktionalität auch mittelbar, durch die Verknüpfung mit einem weiteren Trackinggerät (zum Beispiel ExacTrac der Firma BrainLAB AG, Kirchheim/Heimstetten) erfolgen. Dazu wird zeitgleich zur magnetischen Messung eine physiologische Veränderung des Patienten gemessen, zum Beispiel die Bewegung von Thorax und Bauchwand während der Atmung. Durch Korrelation dieser beiden Signale ist es zum Beispiel auch zu einem späteren Zeitpunkt möglich die folgende Bestrahlung ausschließlich anhand des Signals des zweiten Trackingsystems zu steuern.

### Magnettracking mit Implantat zur atemgetriggerten diagnostischen Bildaufnahme

Die Aufnahmen der diagnostischen Bilder auf denen die Behandlungen basieren wird erfindungsgemäß ähnlich gelöst. Um Artefakte in den Bildern und das Verzerren geometrischer Proportionen in den Datensätzen zu vermeide ist es notwendig, dass alle Bilddaten möglichst gut zu einer Lungenfüllung des Patienten passen. Werden alle Schichtbilder zu Zeitpunkten mit gleicher Lungenfüllung des Patienten aufgenommen, so ist sichergestellt, dass jeder Punkt im Inneren des Patienten nur in genau einem Schichtbild abgebildet wird. Um dies zu erreichen wird die Position des Implantats am bildgebenden Gerät (vorzugsweise CT) kontinuierlich verfolgt, und die Aufnahme eines Schichtbildes nur zu Zeitpunkten gestartet, an denen die Position des Implantats im Patienten innerhalb eines vorgegebenen Toleranzbereiches um eine zuvor festgelegte Position liegt.

### Magnettracking mit Implantat zur Positionierungsvorbereitung außerhalb der Behandlungsposition

Kritisch für magnetisches Tracking sind Störfelder, sowie metallische und andere elektrisch leitende Gegenstände die die magnetischen Felder verzerren. Lässt sich im Umfeld der Therapiemaschine trotz aller Maßnahmen keine Messung mit ausreichender Genauigkeit durchführen, so liefert die vorliegende Erfindung eine alternative Lösung:

Durch die Kombination des magnetischen Trackingsystems mit einem weiteren, zum Beispiel optischen, 3D-Trackinsystem welches die Position der Messpunkte im Raum bestimmen kann, ist es möglich die Position des Implantats in einem Raum oder einem Bereich eines Raumes zu messen, in dem weniger Störquellen vorhanden sind. Eine mögliche Implementierung dieser Vorgehensweise wird im Folgenden anhand des ExacTrac™ -systems der BrainLAB AG (Kirchheim/Heimstetten) erklärt: Die elektromagnetischen Messpunkte stehen in einer bekannten räumlichen Relation zu Referenzpunkten die vom Trackingsystem erfasst werden. In diesem Beispiel befinden sich reflektierende Kugeln auf einer festen Struktur, die fest am Patienten, oder der Liege auf der der Patient liegt, befestigt sind. Wird nun die Position des Implantats relativ zu den Messpunkten bestimmt, so lässt sich über die bekannte räumliche Relation der reflektierenden Kugeln zu diesen Messpunkten die Position des Implantats relativ zu den reflektierenden Kugeln bestimmen.

Wird der Patient nun zum Therapiegerät gebracht, so ist es zwingend notwendig diese räumliche Beziehung nicht zu verändern. Dies lässt sich vorzugsweise dadurch realisieren, dass der Patient mitsamt der Liege auf der er gelagert ist zum Therapiegerät gebracht wird. Zusätzlich kann der Patient noch auf dieser Liege fixiert werden.

Liegt der Patient auf dem Patiententisch der Therapiemaschine wird die Position der reflektierenden Kugeln in Echtzeit erfasst. Da die räumlichen Beziehungen zwischen Implantat und Zielvolumen im Patienten, sowie zwischen Implantat und den reflektierenden Kugeln bekannt sind, kann der Patient nun anhand der reflektierenden Kugeln so positioniert werden, dass das Zielvolumen im Patienten relativ zur Therapiemaschine richtig positioniert ist.

### Magnettracking mit Implantat zur atemgetriggerten Behandlungsvorbereitung außerhalb der Behandlungsposition

Auch für atemgetriggerte Behandlungen kann es von Vorteil oder nötig sein die magnetischen Messungen abseits der Therapiemaschine zu tätigen. Zusätzlich zum Vorteil, dass störende Felder und Signale vermieden werden können, lässt sich die Zeit die der Patient an der Therapiemaschine verbringt dadurch verringern. Wie bereits weiter oben beschrieben wird dazu zeitgleich zur magnetischen Messung eine physiologische Veränderung des Patienten gemessen, zum Beispiel die Bewegung von Thorax und Bauchwand während der Atmung. Durch Korrelation dieser beiden Signale ist es möglich ein Kriterium für das unabhängige Messsystem festzulegen, welches sicher stellt, dass sich das Implantat in der richtigen Position im Patienten befindet.

Wird der Patient im Folgenden zum Therapiegerät gebracht, so kann die Bestrahlung ausschließlich anhand des Signals des zweiten Trackingsystems erfolgen, welches ebenfalls am Therapiegerät vorhanden sein muss.

## Patentansprüche

1. Verfahren zur Positionierung eines Patienten bzw. zur Zielvolumenerfassung in der Strahlentherapie/Radiochirurgie, bei dem mindestens ein Implantat in der Umgebung des Behandlungsziels positionell referenziert bzw. registriert wird, wobei mindestens ein Implantat mittels eines elektromagnetischen Feldes induktiv angeregt wird, die Abstrahlung mindestens eines Implantats erfasst und zur Positions- bzw. Orientierungsbestimmung des Implantats verwendet wird, um hieraus die aktuelle Position des Zielvolumens zu bestimmen, und wobei die Position des Messpunkts (der Messpunkte) an dem (denen) das Signal erfasst wird, relativ zum Therapiegerät bekannt ist (sind), **dadurch gekennzeichnet, dass** ein oder mehrere Messpunkte auf einer festen, mobilen Struktur angebracht sind, deren Position relativ zum Therapiegerät mittels eines Echtzeit-Trackingsystems im dreidimensionalen Raum verfolgt wird.

2. Verfahren nach Anspruch 1, bei dem:
- die Position des Implantats vor der Strahlenbehandlung durch ein bildgebendes System erfasst wird, und relativ zu inneren Organen oder anderen Köperstrukturen in Referenz gesetzt wird,
- der Patient nach der Bildgebung an das Therapiegerät verbracht wird,
- am Therapiegerät ein dynamisches elektromagnetisches Feld in der Nähe, aber außerhalb des Patienten erzeugt wird,
- das Implantat durch das elektromagnetische Feld induktiv Energie aufnimmt,
- das Implantat die aufgenommene Energie zumindest teilweise in Form eines zweiten elektromagnetischen Signals wieder abstrahlt,
- das zweite elektromagnetische Signal außerhalb des Patienten wieder erfasst wird und damit auf die Position und/oder Orientierung des Implantats relativ zu den Messpunkten an denen das Signal erfasst, geschlossen wird,
- die Kenntnis der Position des Implantats in Verbindung mit der Kenntnis der Position innerer Organe des Patienten relativ zum Implantat, dazu genutzt wird, auf die aktuelle Position des Zielvolumens im Patienten zu schließen,
wobei die oben aufgeführten Schritte einzeln oder in jedweder Kombination durchgeführt werden können.

3. Verfahren nach Anspruch 1, bei dem
- die Position des Implantats vor der Strahlenbehandlung durch ein bildgebendes System erfasst wird, und relativ zu inneren Organen oder Köperstrukturen in Referenz gesetzt wird,
- der Patient nach der Bildgebung an das Therapiegerät verbracht wird,
- am Therapiegerät ein dynamisches elektromagnetisches Feld in der Nähe, aber außerhalb des Patienten erzeugt wird,
- das Implantat durch das elektromagnetische Feld induktiv Energie aufnimmt,
- das Implantat die aufgenommene Energie zumindest teilweise in Form eines elektromagnetischen Signals wieder abstrahlt,
- das elektromagnetische Signal außerhalb des Patienten wieder erfasst wird und damit auf die Position und/oder Orientierung des Implantats relativ zu den Messpunkten an denen das Signal erfasst, geschlossen wird,
- die Position des Messpunkts (der Messpunkte) an dem (denen) das Signal erfasst wird relativ zum Therapiegeräts bekannt ist (sind),
- die Kenntnis der Position des Implantats in Verbindung mit der Kenntnis der Position innerer Organe des Patienten relativ zum Implantat, dazu genutzt wird auf die aktuelle Position des Zielvolumens im Patienten zu schließen,
- die Positionserfassung des Implantats fortlaufend oder wiederholt durchgeführt wird, und daraus die atembedingte Verschiebung des Zielvolumens ermittelt wird,
wobei die oben aufgeführten Schritte einzeln oder in jedweder Kombination durchgeführt werden können.

4. Verfahren nach Anspruch 2, bei dem die Messpunkte sich auf dem rotierenden Teil eines Linearbeschleunigers, insbesondere auf dessen Gantry oder Multileaf-Colimator befinden.

5. Verfahren nach Anspruch 2, bei dem die Messpunkte in die Behandlungsliege des Therapiegerätes integriert sind.

6. Verfahren nach Anspruch 2, bei dem das Implantat eine oder mehrere Spulen enthält.

7. Verfahren nach Anspruch 8, bei dem das Implantat mehrere Spulen enthält, deren Achsen nicht parallel zueinander sind.

8. Verfahren nach Anspruch 8 oder 9, bei dem die Spulen im Implantat an verschiedene Schwingkreise mit unterschiedlichen Resonanzfrequenzen angeschlossen sind.

9. Verfahren nach Anspruch 1, bei dem
- der Patient sich während des Trackings des Implantats in einem Raum oder Bereich eines Raumes befindet in dem sich so wenig Störfelder wie möglich befinden, und in dem so wenig metallische Teile in der Nähe des Messortes befinden wie möglich,
- die Position des Implantats relativ zu den Messpunkten bestimmt wird,
- die Messpunkte fest mit dem Patienten oder der Liege auf der der Patient liegt verbunden sind,
- die Messpunkte mit einem Referenzmittel ausgestattet sind welches es erlaubt die Position der Messpunkte mit einem unabhängigen, dreidimensionalen Tracking-system zu bestimmen,
- der Patient nach der elektromagnetischen Messung so bis an das Therapiegerät gebracht wird, dass die räumliche Beziehung zwischen Patient und Messpunkten nicht verändert wird, und
- der Patient anhand des Referenzmittels relativ zum Therapiegerät positioniert wird.

10. Verfahren nach Anspruch 9, bei dem das unabhängige, dreidimensionale Tracking-system ein optisches Infrarot-Kamerasystem ist.

11. Verfahren nach Anspruch 3, bei dem
- mindestens einer der genannten Schritte außerhalb der Behandlungsposition, insbesondere in einem Nebenraum durchgeführt,
- zusätzlich ein Tracking-System die Bewegung und Position von externen Markierungen, zum Beispiel Infrarotmarkern verfolg, wobei die Position und Bewegung des Implantats zur Position und Bewegung der externen Markierungen referenziert wird, und wobei die Positionierung und ein Gating bzw. eine Strahlnachführung basierend lediglich auf der Verfolgung der externen Markierungen durchgeführt wird.

12. Verfahren nach Anspruch 4, bei dem
- am bildgebenden System ein dynamisches elektromagnetisches Feld in der Nähe, aber außerhalb des Patienten erzeugt wird,
- das Implantat durch das elektromagnetische Feld induktiv Energie aufnimmt,
- das Implantat die aufgenommene Energie zumindest teilweise in Form eines elektromagnetischen Signals wieder abstrahlt,
- das elektromagnetische Signal außerhalb des Patienten wieder erfasst wird und damit auf die Position und/oder Orientierung des Implantats relativ zu den Messpunkten an denen das Signal erfasst, geschlossen wird,
- die Position des Messpunkts (der Messpunkte) an dem (denen) das Signal erfasst wird relativ zum bildgebenden System bekannt ist (sind),
- die Kenntnis der Position des eingebrachten Implantats dazu genutzt wird, um das bildgebende System nur zu den Zeitpunkten Daten aufzeichnen zu lassen, zu denen die Position des Implantats innerhalb eines toleranzbehafteten Bereiches innerhalb des Patienten liegt,
wobei die oben aufgeführten Schritte einzeln oder in jedweder Kombination durchgeführt werden können.

## Claims

1. A method for positioning a patient and/or for detecting a target volume in radiotherapy/radiosurgery, wherein at least one implant is positionally referenced or registered in the vicinity of the treatment target, at least one implant is inductively stimulated by means of an electromagnetic field, and the emission from at least one implant is detected and used to determine the position and/or orientation of the implant, in order to determine from this the current position of the target volume, and wherein the position of the measuring point(s) at which the signal is detected is/are known relative to the therapy device, **characterised in that** one or more measuring points are attached to a solid, mobile structure whose position relative to the therapy device is tracked three-dimensionally by means of a real-time tracking system.

2. The method according to claim 1, wherein:
- the position of the implant is detected using an imaging system before the radiation treatment, and referenced relative to inner organs or other body structures;
- after imaging, the patient is moved to the therapy device;
- at the therapy device, a dynamic electromagnetic field is generated in the vicinity of but outside of the patient;
- the implant inductively absorbs energy via the electromagnetic field;
- the implant at least partially re-emits the absorbed energy in the form of a second electromagnetic signal;
- the second electromagnetic signal is again detected outside the patient, and the position and/or orientation of the implant relative to the measuring points at which the signal is detected is thus deduced;
- knowledge of the position of the implant, in combination with knowledge of the position of the patient's inner organs relative to the implant, is used to deduce the current position of the target volume within the patient;
wherein the steps quoted above can be performed individually or in any combination.

3. The method according to claim 1, wherein:
- the position of the implant is detected using an imaging system before the radiation treatment, and referenced relative to inner organs or other body structures;
- after imaging, the patient is moved to the therapy device;
- at the therapy device, a dynamic electromagnetic field is generated in the vicinity of but outside of the patient;
- the implant inductively absorbs energy via the electromagnetic field;
- the implant at least partially re-emits the absorbed energy in the form of an electromagnetic signal;
- the electromagnetic signal is again detected outside the patient, and the position and/or orientation of the implant relative to the measuring points at which the signal is detected is thus deduced;
- the position of the measuring point(s) at which the signal is detected is/are known relative to the therapy device;
- knowledge of the position of the implant, in combination with knowledge of the position of the patient's inner organs relative to the implant, is used to deduce the current position of the target volume within the patient;
- the position of the implant is detected continuously or repeatedly and from this, the shift of the target volume caused by breathing is determined;
wherein the steps quoted above can be performed individually or in any combination.

4. The method according to claim 2, wherein the measuring points are situated on the rotating part of a linear accelerator, in particular on its gantry or multi-leaf collimator.

5. The method according to claim 2, wherein the measuring points are integrated into the treatment couch of the therapy device.

6. The method according to claim 2, wherein the implant contains one or more coils.

7. The method according to claim 6, wherein the implant contains a number of coils whose axes are not parallel to each other.

8. The method according to claim 6 or 7, wherein the coils in the implant are connected to different oscillating circuits having different resonance frequencies.

9. The method according to claim 1, wherein:
- while the implant is tracked, the patient is situated in a space or region of a space in which there are as few interference fields as possible and in which there are as few metallic parts in the vicinity of the measuring site as possible;
- the position of the implant relative to the measuring points is determined;
- the measuring points are fixedly connected to the patient or to the couch on which the patient is lying;
- the measuring points are fitted with a reference means which allows the position of the measuring points to be determined using an independent, three-dimensional tracking system;
- after electromagnetic measuring, the patient is moved to the therapy device in such a way that the spatial relationship between the patient and the measuring points is not changed; and
- the patient is positioned relative to the therapy device by way of the reference means.

10. The method according to claim 9, wherein the independent, three-dimensional tracking system is an optical infrared camera system.

11. The method according to claim 3, wherein:
- at least one of the steps cited is performed outside the treatment position, in particular in an adjacent space;
- a tracking system additionally tracks the movement and position of external markings, for example infrared markers, wherein the position and movement of the implant is referenced with respect to the position and movement of the external markings, and
wherein positioning, gating and/or beam tracking are based only on tracking the external markings.

12. The method according to claim 4, wherein:
- at the imaging system, a dynamic electromagnetic field is generated in the vicinity of but outside of the patient;
- the implant inductively absorbs energy via the electromagnetic field;
- the implant at least partially re-emits the absorbed energy in the form of an electromagnetic signal;
- the electromagnetic signal is again detected outside the patient, and the position and/or orientation of the implant relative to the measuring points at which the signal is detected is thus deduced;
- the position of the measuring point(s) at which the signal is detected is/are known relative to the imaging system;
- knowledge of the position of the introduced implant is used to cause the imaging system to record data only when the position of the implant is within a tolerance range within the patient;
wherein the steps quoted above can be performed individually or in any combination.

## Revendications

1. Procédé pour positionner un patient ou détecter un volume cible en radiothérapie/radiochirurgie, dans lequel on repère la position d'au moins un implant dans l'environnement de la cible de traitement, au moins un implant étant excité de manière inductive au moyen d'un champ électromagnétique, le rayonnement d'au moins un implant est relevé et utilisé pour déterminer la position ou l'orientation de l'implant, afin de déterminer à partir de ceci la position actuelle du volume cible, et la position du point de mesure (des points de mesure) auquel (auxquels) le signal est détecté, est connue par rapport à l'appareil de thérapie, **caractérisé en ce que** l'on place un ou plusieurs points de mesure sur une structure fixe, mobile dont la position par rapport à l'appareil de thérapie est suivie au moyen d'un système de poursuite en temps réel, dans l'espace tridimensionnel.

2. Procédé selon la revendication 1 dans lequel :
- on relève la position de l'implant avant le traitement par radiation par un système d'imagerie, et on la repère par rapport à des organes ou à d'autres structures corporelles,
- on amène le patient à l'appareil de thérapie après acquisition de l'image,
- on produit sur l'appareil de thérapie un champ électromagnétique dynamique à proximité, mais à l'extérieur du patient,
- l'implant reçoit de l'énergie par le champ électromagnétique,
- l'implant ré-émet l'énergie absorbée au moins en partie sous la forme d'un second signal électromagnétique,
- on détecte à nouveau le second signal électromagnétique à l'extérieur du patient et on conclut ainsi à la position et/ou à l'orientation de l'implant par rapport aux points de mesure auxquels le signal est détecté,
- on utilise la connaissance de la position de l'implant en combinaison avec la connaissance de la position d'organes internes du patient par rapport à l'implant, afin de conclure à la position actuelle du volume cible dans le patient,
les étapes indiquées ci-dessus pouvant être exécutées séparément ou dans n'importe quelle combinaison.

3. Procédé selon la revendication 1 dans lequel
- on relève la position de l'implant avant le traitement par radiation par un système d'imagerie, et on la repère par rapport à des organes internes ou des structures corporelles,
- on amène le patient à l'appareil de thérapie après acquisition de l'image,
- on produit sur l'appareil de thérapie un champ électromagnétique dynamique à proximité, mais à l'extérieur du patient,
- l'implant reçoit de l'énergie par le champ électromagnétique,
- l'implant ré-émet l'énergie absorbée au moins en partie sous la forme d'un signal électromagnétique,
- on détecte à nouveau le signal électromagnétique à l'extérieur du patient et on conclut ainsi à la position et/ou à l'orientation de l'implant par rapport aux points de mesure auxquels le signal est détecté,
- la position du point de mesure (des points de mesure) auquel (auxquels) le signal est détecté est connue par rapport à l'appareil de thérapie,
- on utilise la connaissance de la position de l'implant en combinaison avec la connaissance de la position d'organes internes du patient par rapport à l'implant, afin de conclure à la position actuelle du volume cible dans le patient,
- on exécute en continu ou de manière renouvelée le relevé de la position de l'implant et on détermine à partir de celui-ci le déplacement, dû à la respiration, du volume cible,
les étapes indiquées ci-dessus pouvant être exécutées séparément ou dans n'importe quelle combinaison.

4. Procédé selon la revendication 2, dans lequel les points de mesure se trouvent sur la partie rotative d'un accélérateur linéaire, en particulier sur son portique ou collimateur multilame.

5. Procédé selon la revendication 2, dans lequel les points de mesure sont intégrés dans le lit de traitement de l'appareil de thérapie.

6. Procédé selon la revendication 2, dans lequel l'implant contient une ou plusieurs bobines.

7. Procédé selon la revendication 6, dans lequel l'implant contient plusieurs bobines dont les axes ne sont pas parallèles entre eux.

8. Procédé selon l'une quelconque des revendications 6 ou 7, dans lequel les bobines de l'implant sont connectées à différents circuits oscillants avec des fréquences de résonance différentes.

9. Procédé selon la revendication 1, dans lequel
- pendant la poursuite de l'implant le patient se trouve dans une pièce ou zone d'une pièce dans laquelle se trouvent aussi peu de champs parasites que possible, et dans laquelle aussi peu que possible de pièces métalliques se trouvent à proximité de l'emplacement de mesure,
- on détermine la position de l'implant par rapport aux points de mesure,
- les points de mesure sont reliés de manière fixe au patient ou au lit sur lequel se trouve le patient,
- les points de mesure sont équipés d'un moyen de référence qui permet de déterminer la position des points de mesure avec un système de poursuite tridimensionnel, indépendant,
- après la mesure électromagnétique, on amène le patient à l'appareil de thérapie de manière que la relation spatiale entre le patient les points de mesure ne soit pas modifiée, et
- on positionne le patient par rapport à l'appareil de thérapie, à l'aide du moyen de référence.

10. Procédé selon la revendication 9, dans lequel le système de poursuite tridimensionnel, indépendant, est un système optique de caméra à infrarouge.

11. Procédé selon la revendication 3, dans lequel
- on exécute au moins l'une des étapes citées à l'extérieur de la position de traitement, en particulier dans une pièce auxiliaire,
- en supplément, un système de poursuite suit le mouvement et la position de repères externes, par exemple de repères infrarouges, la position et le mouvement de l'implant étant repérés par rapport à la position et au mouvement des repères externes, et on procède au positionnement et à un gating ou à une localisation du faisceau uniquement sur la base de la poursuite des repères externes.

12. Procédé selon la revendication 4, dans lequel
- on produit sur le système d'imagerie un champ électromagnétique dynamique à proximité, mais à l'extérieur du patient,
- l'implant reçoit de l'énergie de manière inductive par le champ électromagnétique,
- l'implant ré-émet l'énergie absorbée au moins en partie sous la forme d'un signal électromagnétique,
- on détecte à nouveau le signal électromagnétique à l'extérieur du patient et on conclut ainsi à la position et/ou à l'orientation de l'implant par rapport aux points de mesure auxquels le signal est détecté,
- la position du point de mesure (des points de mesure) auquel (auxquels) le signal est détecté est connue par rapport au système d'imagerie,
- on utilise la connaissance de la position de l'implant introduit, afin de faire en sorte que le système d'imagerie n'enregistre des données qu'aux instants auxquels la position de l'implant se situe à l'intérieur d'une zone avec tolérances, à l'intérieur du patient,
les étapes indiquées ci-dessus pouvant être exécutées séparément ou dans n'importe quelle combinaison.
